# EUROPÄISCHE PATENTSCHRIFT

(11) **EP 2 308 525 B1**
(45) Veröffentlichungstag und Bekanntmachung des Hinweises auf die Patenterteilung: **25.03.2015**
(21) Anmeldenummer: 10011806.6
(22) Anmeldetag: 22.05.2003
(51) Int. Cl.: A61M 1/16, A61M 1/34, A61M 1/36, A61M 1/10

(54) **Vorrichtung zur Behandlung einer medizinischen Flüssigkeit**
Device for the treatment of a medical liquid
Dispositif de traitement pour traiter un fluide médical

(30) Priorität: 04.06.2002 DE 10224750
(43) Veröffentlichungstag der Anmeldung: 13.04.2011
(62) Teilanmeldung aus: 03730095.1
(73) Patentinhaber: Fresenius Medical Care Deutschland GmbH, 61352 Bad Homburg v.d.H. (DE)
(72) Erfinder: Beden, Josef, 55252 Mainz-Kastel (DE); Hahmann, Uwe, 61231 Bad Nauheim (DE); Herklotz, Martin, 63150 Heusenstamm (DE); Lauer, Martin, 66606 St. Wendel (DE); Manke, Joachim, 35792 Löhnberg (DE); Scheunert, Peter, 61381 Friedrichsdorf (DE); Weis, Manfred, 66606 St. Wendel (DE); Bongers, Alexander, 63225 Langen (DE)
(74) Vertreter: Laufhütte, Dieter

(56) Entgegenhaltungen:
- WO-A-84/02473
- US-A- 5 378 126
- US-A- 5 431 627

## Beschreibung

### Beschreibung

Die Erfindung betrifft eine Vorrichtung zur Behandlung einer medizinischen Flüssigkeit mit einer Flüssigkeitsbehandlungsmaschine und einer in diese einsetzbaren Kassette im wesentlichen bestehend aus einem starren Kassettengrundkörper mit eingelassenen Kammern und Kanälen und einer diese abdeckenden Folie.

Entsprechende Kassetten werden in der Medizintechnik eingesetzt, insbesondere um Dialysierflüssigkeit, Blut und dergleichen zu fördern. Kassetten dieser Art werden z. B. in der DE 198 37 667, der WO 84/02473, der WO 98/22165 oder der WO 00/33898 beschrieben.

So ist beispielsweise aus der DE 198 37 667 A1 bereits eine Kassette bekannt, die aus einem Kassettengrundkörper mit eingelassenen Kammern und Kanälen besteht, welche mit einer flexiblen Folie zur Abdeckung der Kanäle und Kammern abgeschlossen ist. Es ist dort bereits beschrieben, daß die Kassette in eine spezielle Aufnahmekammer z. B. in eine Dialysemaschine eingesetzt wird. Diese Kammer kann beispielsweise über eine schwenkbare Tür geöffnet werden. Die Kassette ist in die Kammer einlegbar, wobei der flexiblen Folie ein entsprechendes Gegenstück an der Maschine gegenüberliegt, damit die Kassette mit Hilfe von maschinenseitigen Aktoren und Sensoren betrieben werden kann.

Obwohl bereits derartige Vorrichtungen mit Kassetten grundsätzlich beschrieben sind, liegen die herkömmlichen extrakorporalen Blutkreisläufe bzw. Blutschlauchsysteme üblicherweise in Differentialbauweise vor. Das heißt, daß eine Funktionsaufteilung auf verschiedene Komponenten vorliegt. Solche Komponenten, beispielsweise Blasenfänger, Flußkammern oder Zuspritzstellen, sind durch Schläuche miteinander verbunden und werden in der Regel einzeln mit der jeweiligen Dialysemaschine verbunden. Der Aufbau derartiger Blutschlauchsysteme ist nun in der Herstellung und Handhabung sehr aufwendig, wobei der entsprechende Aufwand selbstverständlich bei komplexeren Systemen, wie einer online-Hämodiafiltration äußerst zeitraubend sein kann.

Dafür haben die herkömmlichen extrakorporalen Blutkreisläufe, die in dieser Differentialbauweise aufgebaut sind, den Vorteil, daß sie je nach Anforderung für die jeweilige Behandlung wesentlich flexibler gestaltbar sind. Den bisher bekannten Vorrichtungen zum Einsatz von Kassetten haftete nämlich das Problem an, daß diese nur für einen ganz bestimmten Anwendungsfall einsetzbar sind.

Die WO 84/02473 A1 offenbart eine Vorrichtung zur Behandlung einer medizinischen Flüssigkeit mit einer Flüssigkeitsbehandlungsmaschine und einer in dieser einsetzbaren Kassette. Die Kassette besteht im Wesentlichen aus einem starren Grundkörper mit eingelassenen Kammern und Kanälen und eine fluidundurchlässigen Folie, die diese abdeckt.

Die US 5,378,126 offenbart eine Pumpenanordnung, insbesondere eine Infusionspumpe, die einen Kalottenantrieb aufweist. Diese Pumpenanordnung ist aber nicht geeignet ist, als eine Blutpumpe eingesetzt zu werden.

Aufgabe der vorliegenden Erfindung ist es daher, eine gattungsgemäße Vorrichtung mit einer Flüssigkeitsbehandlun smaschine und einer in diese einsetzbaren Kassette derart weiterzubilden, daß das Pumpen der Flüssigkeitschonend erfolgt.

Diese Aufgabe wird erfindungsgemäß mittels einer Vorrichtung nach der Merkmalskombination des Anspruchs 1 gelöst.

Die erfindungsgemäße Vorrichtung zur Behandlung einer medizinischen Flüssigkeit weist eine in diese einsetzbare Kassette auf, die im Wesentlichen aus einem staren Kassettengrundkörper mit eingelassenen Kammern und Kanälen und einer diese abdeckenden Folie besteht. Erfindungsgemäß weist wenigstens eine der Kammern der Kassette eine Pumpkammer zum Pumpen von Blut auf, wobei die mindestens eine Pumpkammer im Wesentlichen die Form eines Kugelabschnittes hat und wobei um die Kammer herum am Rand ein hochstehender Wulst ausgeformt und der umlaufende Rand des Kugelabschnittes etwas tiefer gesetzt ist, so dass sich in der Ausdrückphase zwischen dem oberen Rand des Kassettengrundkörpers und der Deckfolie ein Spülkanal ausbildet. Durch die erfindungsgemäße Merkmalskombination wird erreicht, dass eine Pumpkammer zum Pumpen von Blut vorteilhaft gespült werden kann und zugleich, dass Blut schonend gepumpt werden kann.

Vorteilhafterweise werden in einer gattungsgemäßen Vorrichtung zur Behandlung einer medizinischen Flüssigkeit Aktoren und Sensoren zum Betrieb der Vorrichtung mit einer eingesetzten Kassette derart angeordnet, daß Kassetten in verschiedenen Integrationsformen einsetzbar sind.

Aufgrund der eindeutig definierten Anordnung entsprechender Sensoren und Aktoren können unterschiedlich komplexe Kassetten entsprechend dem gewünschten Anwendungsfall in die Flüssigkeitsbehandlungsmaschine eingesetzt werden. Es ist also hier nicht notwendig, unterschiedliche Vorrichtungen für unterschiedliche Anwendungsfälle bereitszustellen.

So kann hier beispielsweise eine Kassette für eine einfache Standard-Hämodialyse einsetzbar sein. Die entsprechenden Pumpkammern, Meßsensoren und weiteren Aktoren, wie Ventile etc. sind hier in der Flüssigkeitsbehandlungsmaschine an vorbestimmten Orten vorgesehen. Es sind noch zusätzliche Pumpen, Aktoren, Ventile etc. in der Flüssigkeitsbehandlungsmaschine vorgesehen, die bei Einsatz der Kassette für die Standard-Hämodialyse nicht zu betätigen sind. Diese sind beispielsweise erst bei Einsatz einer Kassette für eine online-Hämodiafiltration oder eine online-Hämofiltration im Einsatz, d. h. in den entsprechenden Kassetten sind weitere Kanäle, Pumpkammern etc. an entsprechenden Stellen vorgesehen, die diesen Aktoren, Pumpen oder Ventilen zugeordnet sind. Weiterhin läßt sich eine Kassette für eine Akut-Dialysebehandung einsetzen, bei der wiederum die seitens der Flüssigkeitsbehandlungsmaschine vorgesehenen Pumpen, Aktoren und Ventile entsprechenden Pumpkammern, Kanälen etc. zugeordnet werden. Die zugehörige Steuerelektronik ist je nach eingesetzter Kassette zur Ansteuerung der Pumpen, Aktoren, Sensoren etc. auswählbar.

Besonders vorteilhafte Ausgestaltungen der Erfindung ergeben sich aus den sich an den Hauptanspruch anschließenden Unteransprüchen 2 bis 8. Erfindungsgemäße Kassetten zum Einsatz in die vorgenannte erfinderische Vorrichtung ergeben sich in besonderer Ausgestaltung aus den sich anschließenden Ansprüchen 9 bis 15.

Außerdem ist Gegenstand der Erfindung ein Kassettenset mit den Merkmalen des Anspruchs 16.

Einzelheiten und Vorteile der Erfindung werden anhand der in Anlage beigefügten Figuren im folgenden beispielhaft erläutert. Es zeigen:
- Fig. 1:: eine schematische Draufsicht auf eine Kassette für eine Standard-Hämodialyse,
- Fig. 2:: eine schematische Draufsicht auf eine erfindungsgemäße Kassette gemäß einer weiteren Ausführungsform der Erfindung zur Verwendung bei der online-Hämodiafiltration oder online-Hämofiltration,
- Fig. 3:: eine Draufsicht auf eine Kassette gemäß einer weiteren Ausführungsform der vorliegenden Erfindung, die für die Akut-Behandlung einsetzbar ist,
- Fig. 4:: eine schematische Draufsicht auf eine weitere Ausgestaltung der Erfindung, die im wesentlichen derjenigen gemäß Fig. 1 entspricht, jedoch einen integrierten Dialysator aufweist,
- Fig. 5:: eine weitere Ausgestaltung der Erfindung, die im wesentlichen derjenigen gemäß Fig. 2 entspricht, jedoch einen integrierten Dialysator aufweist,
- Fig. 6:: eine weitere Ausführungsform der Erfindung, die im wesentlichen derjenigen gemäß Fig. 3 entspricht, jedoch einen integrierten Dialysator aufweist,
- Fig. 7:: eine dreidimensionale Darstellung einer Flüssigkeitsbehandlungsmaschine als Ausführungsform der erfindungsgemäßen Vorrichtung ohne eingesetzte Kassette,
- Fig. 8:: eine Darstellung entsprechend Fig. 7, jedoch mit einer eingesetzten Kassette,
- Fig. 9:: eine Darstellung gemäß Fig. 7, jedoch mit einer sich von der in Fig. 8 dargestellten Kassette unterscheidenden anderen Ausführungsvariante einer Kassette,
- Fig. 10:: ein Detail an einer Entlüftungseinheit in der erfindungsgemäßen Vorrichtung,
- Fig. 11:: eine Detailansicht einer Kontur einer Meßkammer in einer Kassette gemäß einer der vorgenannten Ausführungsvarianten,
- Fig. 12:: eine teilweise Schnittdarstellung einer Pumpkammer der Kassette nach der vorliegenden Erfindung und
- Fig. 13:: eine teilweise Schnittdarstellung durch einen Kanal der Kassette gemäß einer Ausführungsvariante der Erfindung.

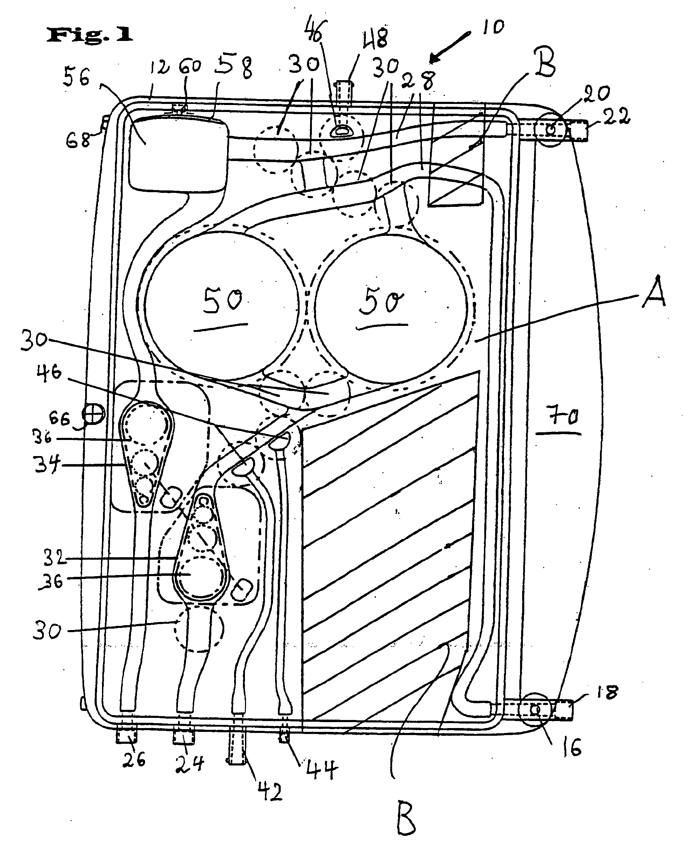

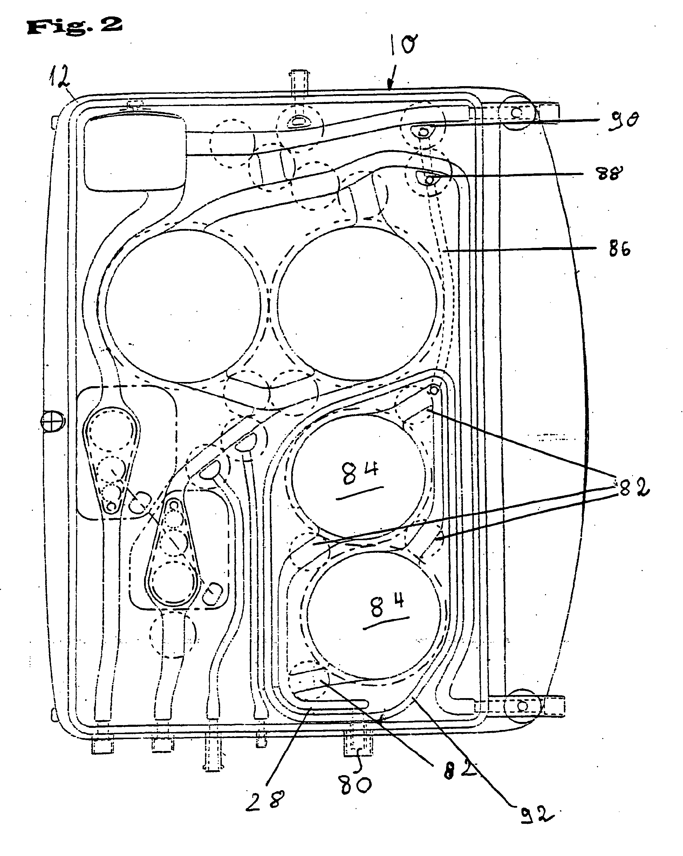

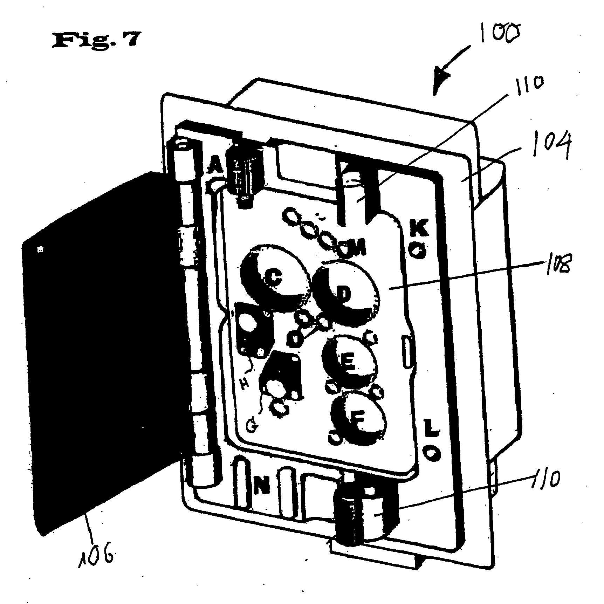

In Fig. 1 ist eine Kassette 10 entsprechend einer Ausführungsvariante der vorliegenden Erfindung dargestellt, die in dieser Ausführung für die Standard-Hämodialyse einsetzbar ist. In Fig. 1 ist die Oberfläche der Kassette 10 in einen schraffierten Bereich B (zwei Teilflächen) und einen nicht schraffierten Bereich A unterteilt. Sowohl die Oberfläche der Kassette 10 wie auch die Oberfläche des zugehörigen Maschinenblocks (vgl. Fig. 7) sind in diese sich überdeckende Flächenbereiche A und B unterteilt, wobei im Flächenbereich A (nicht schraffiert in Fig. 1) die Komponenten von anzukoppelnden Aktoren bzw. Sensoren untergebracht sind, die als Basisvariante allen Kassetten gemeinsam sind, beispielsweise auch den hier dargestellten Kassetten für die Standard-Hämodialyse, und wobei die Flächen B Bereiche angeben, in denen optional zu verwendende Aktoren bzw. Sensoren im Maschinenblock (vgl. Fig. 7) vorgesehen sind, die nur bei Bedarf eingesetzt werden, beispielsweise bei Kassetten entsprechend der Fig. 2.

Die Kassette besteht aus einem Kassettengrundkörper 12, der im hier dargestellten Ausführungsbeispiel aus Polypropylen besteht. Auf dem Kassettengrundkörper ist eine hier nicht näher dargestellte Deckfolie, die beispielsweise aus einem Polyolefin-Elastomer-Gemisch besteht, aufgebracht. Über diese Deckfolie 14 werden die Kanäle und Vertiefungen, auf die später eingegangen werden wird, abgedeckt. Es ist ein arterielles Zuspritzseptum 16 in der arteriellen Überleitung 18 zum Dialysator, sowie ein venöses Zuspritzseptum 20 in der venösen Überleitung 22 zum Dialysator vorgesehen. Der Dialysator selbst und die entsprechende Schlauchverbindung sind im hier dargestellten Ausführungsbeispiel nicht näher gezeigt. Mit 24 ist der Bluteingang vom Patienten und mit 26 der Blutausgang zum Patienten bezeichnet. Auch hier sind die jeweiligen Schläuche, die ebenfalls aus einem Polyolefin-Elastomer-Gemisch bestehen, aus Vereinfachungsgründen nicht dargestellt. Im Kassettengrundkörper 12 sind Kanäle 28 ausgenommen. Diese werden durch eine Reihe von Ventilen 30 beaufschlagt.

Der Aufbau dieser Ventile ergibt sich beispielsweise aus der deutschen Patentanmeldung DE 100 46 651 der Anmelderin, auf die diesbezüglich verwiesen wird. Im wesentlichen weisen diese Ventile 30 einen Ventilkörper mit einem Druckkanal und einer Dichtkappe auf, die mit dem Ventilkörper derart zusammenwirkt, daß sie das ventilkörperseitige Ende des Druckkanals gegen die Umgebung abschließt, wobei zwischen dem Druckkanal und der Dichtkappe ein Druckraum aufbaubar ist, so daß die Dichtkappe einen verformbaren Dichtbereich zum Eintritt in den Fluidkanal aufweist, um diesen ggf. zu verschließen.

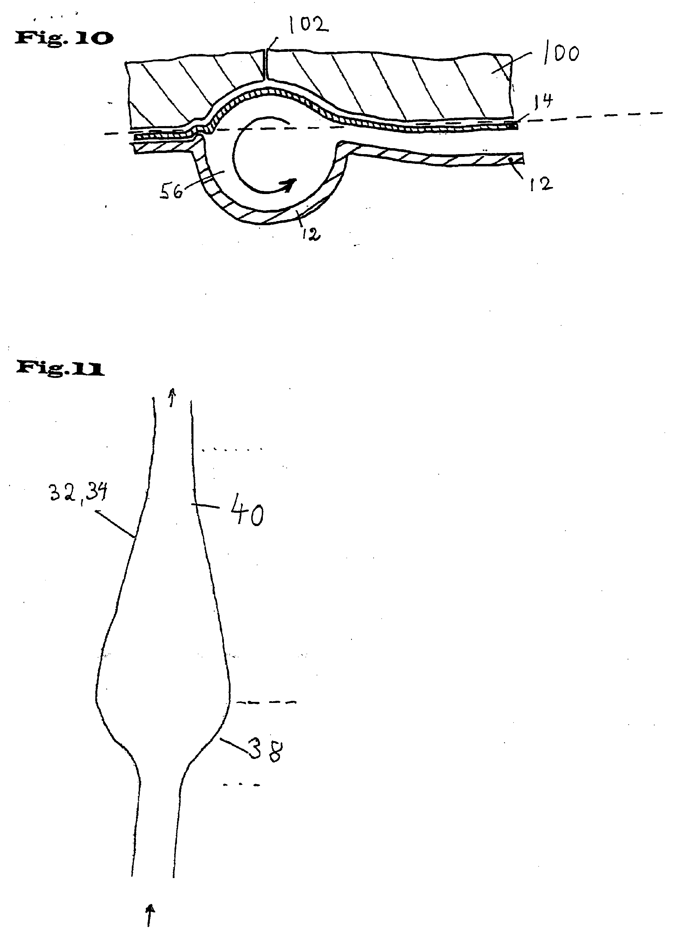

Im Kassettengrundkörper 12 sind weiterhin eine arterielle Meßkammer 32 und eine venöse Meßkammer 34 ausgenommen. Der prinzipielle Aufbau dieser Meßkammern ergibt sich beispielsweise aus Fig. 11, in welcher durch die Pfeile die Fließrichtung der Flüssigkeit, d. h. des Blutes angezeigt ist. Die Meßkammern 32 und 34 weisen eine Kanalaufweitung auf, um die Sensoren 36 aufnehmen zu können. Die Kontur der Meßkammern 32, 34 entspricht einer Diffusordüsengeometrie, wie sie in Fig. 11 dargestellt ist. Im Bereich des Einströmbereichs der Flüssigkeit ist ein Diffusor 38 angeordnet, der in einer Düse 40 ausläuft. Dabei ist die Querschnittserweiterung im Diffusor 38 im Vergleich zur Querschnittsverengung in der Düse 40 relativ zügig. Im Bereich der arteriellen bzw. venösen Meßkammer, 32, 34, sind die Sensoren 36 angeordnet, die in Form von Multifunktionssensoren ausgebildet sind. Der Aufbau dieser Multifunktionssensoren ist im Detail in der deutschen Patentanmeldung DE 198 37 667 A1 der Anmelderin ausführlich erläutert. Auf diese Erläuterung wird vollinhaltlich Bezug genommen.

An der Kassette ist ein arterieller Port 42 und ein Heparin-Port 44 vorgesehen, die über entsprechende Kanäle mit dem das arterielle Blut führenden Kanal jeweils über Phantomventile 46 verbunden sind. Die Phantomventile 46 sind in der erfindungsgemäßen Kassette 10 anstelle von herkömmlichen offenen T-Verzweigungen eingesetzt. Bei diesen Phantomventilen ist die Kanalwand aus Sicht der Hauptblutströmung nicht unterbrochen. Der detaillierte Aufbau dieser Phantomventile ergibt sich aus der deutschen Patentanmeldung DE 100 53 441 derselben Anmelderin, auf die hier verwiesen wird. Mit 48 ist ein venöser Port bezeichnet, der ebenfalls über ein Phantomventil 46 in einen Blut führenden Kanal 28 einmündet, hier im venösen Teil der blutführenden Kanäle.

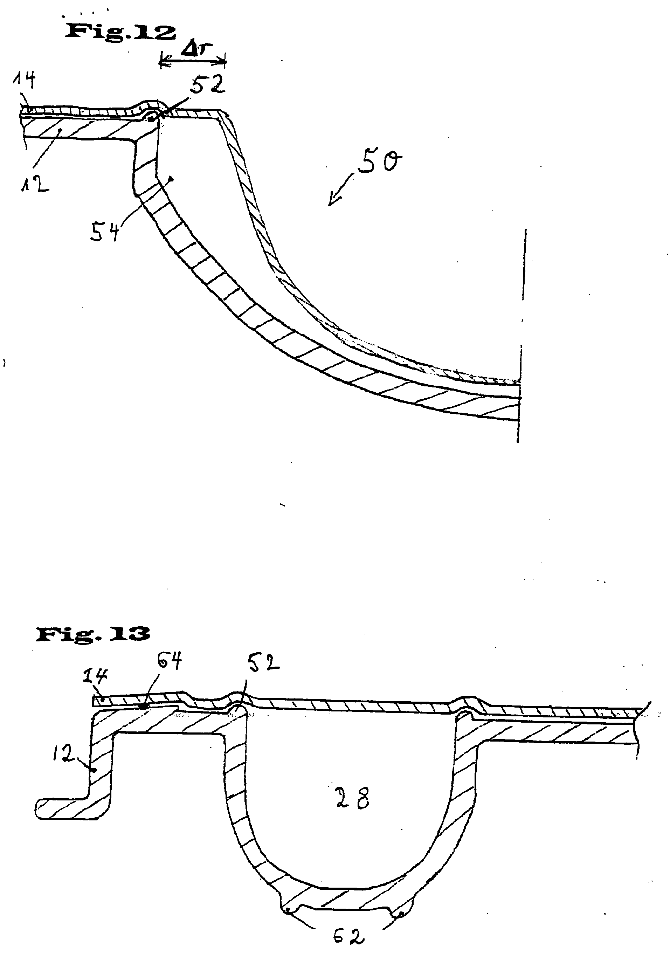

Mit 50 sind zwei Pumpkammern bezeichnet, die zum Pumpen des Blutes dienen. Der Aufbau der Pumpkammern 50 ergibt sich im Detail aus Fig. 12. Die über maschinenseitig vorgesehene Membranpumpen aktivierten Pumpkammern 50 verfügen über im wesentlichen tangentiale Zu- und Abgänge für eine gleichmäßige Durchströmung der gesamten Kammer, wie sich bereits aus der Fig. 1 ergibt. Die Form der Pumpkammern 50 wird durch den entsprechend geformten Kassetten-grundkörper 12 vorgegeben und kann näherungsweise als Kugelabschnitt beschrieben werden. Umlaufend weist der Kassettengrundkörper um die Pumpkammern 50 herum einen als Abpresswulst dienenden erhöhten Rand 52 auf. Zusätzlich ist der umlaufende Rand des Kugelabschnitts etwas tiefer gesetzt, wie sich aus der Fig. 12 ergibt, so daß in der Ausdrückphase, also in der Phase, in welcher die Deckfolie 14 auf den Kassettengrundkörper 12 hinbewegt ist, ein Spülrand - bzw. -kanal 54 gebildet ist. Der Spülrand - bzw. -kanal 54 wird vorteilhaft dadurch ausgebildet, dass die in der Figur 12 nicht dargestellte maschinenseitige Pumpkalotte einen kleineren Radius als der Radius der kassettenseitigen Pumpkammer aufweist. Der Radiusunterschied Δr ist in Figur 12 eingezeichnet. Hierdurch bildet sich ein breiter Spülrand - bzw. -kanal 54 aus. Dieser Spülrand - bzw. -kanal 54 ist ein Ringraum für das gepumpte Blut in der Ausdrück-Extremstellung. Dieser Ringfreiraum vermeidet zum einen die Blutschädigung durch Einklemmen zwischen Folien-und Spritzgussoberfläche am Ende der Ausdrückphase und zum anderen die Blutschädigung durch große Fließgeschwindigkeiten und Scherbeanspruchungen, die sich bei nicht vorgesehenem Ringfreiraum zu Beginn der Anlaufphase ergeben würden.

Im Einbauzustand oberen Bereich der Kassette ist eine Entlüftungskammer 56 ausgebildet, die in Schnittdarstellung nochmals in Fig. 10 dargestellt ist. In dieser Entlüftungskammer ist eine Entlüftungsmembran 58 angeordnet, über die entsprechend gesammelte Luft abgeschieden werden kann, da sie als teildurchlässige Membran, die vorzugsweise hydrophobe oder oleophobe Eigenschaften aufweist, ausgebildet ist. Als Entlüftungsmembran kann vorzugsweise expandiertes oder gesintertes Polytetrafluorethylen eingesetzt werden. Oberhalb der Entlüftungsmembran 58 ist ein Entlüftungsstutzen 60 angeordnet, dessen Zusammenwirkung mit der hier nicht näher dargestellten Flüssigkeitsbehandlungsmaschine später beschrieben wird. In der Entlüftungskammer 56 werden durch eine Verlangsamung des Blutstroms Blasen gefangen. Es wird, wie in Fig. 10 gezeigt, eine Rotationsströmung zur effektiven Luftabscheidung bei minimalem Flächenbedarf auf der Kassette 10 erzeugt. Dabei entsteht die Erzeugung der endgültigen Rotationsströmung erst im Betriebszustand der Kassette 10 in der Flüssigkeitsbehandlungsmaschine 100 (vgl. Fig. 10). Durch ein entsprechendes Vakuumankopplungssystem, von welchem in der Fig. 10 lediglich ein Vakuumsaugkanal 102 dargestellt ist, wird die Deckfolie 14 der Kassette 10 in die Flüssigkeitsbehandlungsmaschine hineingezogen. Dadurch wird ein nahezu kreisförmiger Querschnitt der Entlüftungskammer 56 gebildet. Die Rotationsströmung des Blutes wird dadurch unterstützt, daß auch der in die Entlüftungskammer 56 einmündende Kanal mitsamt seiner Deckfolie 14 leicht in die Maschinenseite hineinläuft, so daß eine nahezu tangentiale Einströmung innerhalb der Kammer erreicht wird. Am Entlüftungsstutzen 60 kann maschinenseitig eine aktive Absaugung erfolgen. Insgesamt ergibt sich hier in der Entlüftungskammer 56 konstruktionsbedingt ein geringes Füllvolumen.

Anhand Fig. 13 kann der prinzipielle Aufbau der Kanäle 28 erläutert werden. Grundsätzlich wird bei der Kanalgestaltung der Kanäle 28 darauf geachtet, daß eine glatte Folienoberfläche und glatte Kanalflächen geschaffen sind. Stufen, Toträume, Turbulenzen und Prallflächen werden vermieden. Es werden geringe Richtungs- und Geschwindigkeitsänderungen angestrebt. Strömungsablösungen werden weitgehend vermieden. Kanalbegleitend haben alle Kanäle 28 und auch Kammern 50 einen Randwulst 52 (vgl. auch Fig. 12), der der Abdeckfolie 14 zugewandt ist. Beim Einsetzen der Kassette 10 in die Flüssigkeitsbehandlungsmaschine 100 wird die Folie 14 auf den Randwulst 52 gedrückt, so daß alle Kanäle 28 gegen die Umgebung abgedichtet sind. Auf der Rückseite der Kassette, d. h. an der Außenseite der Kanalwandung sind kanalbegleitende Stege 62 ausgebildet, über die die rückseitige Verpressungskraft zu den Randwülsten 52 geleitet wird, um so eine gleichmäßige, linienhafte Kraftverteilung zu erreichen.

Anhand der Fig. 13 kann auch erläutert werden, daß am Außenrand 64 der Kassettengrundkörper 12 mit der Deckfolie 14 verschweißt ist.

Die Kassette 10 weist als Positionierhilfe eine ausgenommene Zentriergabel 66 auf, die beim Einsetzen einen maschinenseitigen Zentrierstift aufnimmt. Weiterhin sind Anschlagnasen 68 angeformt, die sich gegen entsprechende Maschinenflächen beim Einlegen anlegen. Dadurch wird die Kassette 10 in Höhe und Winkel geführt. Beim Andrücken der Kassette 10 in die Flüssigkeitsbehandlungsmaschine 100 findet an einem hier nicht näher dargestellten Schnappelement eine Verrastung mit der Flüssigkeitsbehandlungsmaschine statt, so daß die Kassette 10 ausgerichtet fixiert ist. Zur vereinfachten Handhabung weist die Kassette an der der Zentriergabel 66 gegenüberliegenden Seite einen angeformten Handgriff 70 auf.

Das arterielle Zuspritzseptum 16 bzw. das venöse Zuspritzseptum 20 sind im hier dargestellten Ausführungsbeispiel im Gegensatz zu einer herkömmlichen Zuspritzstelle derart ausgebildet, daß deren Grundkörper durch den Kassettengrundkörper 12 selbst gebildet ist, so daß hier nur das elastische Septum durch einen Schnappring (hier nicht näher dargestellt) fixiert ist. Das Septum besteht im hier dargestellten Ausführungsbeispiel aus einem Elastomer.

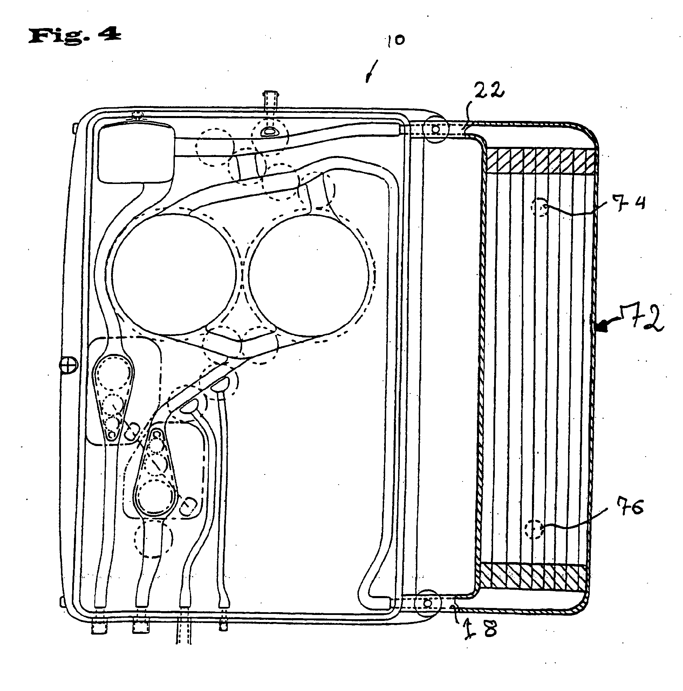

Fig. 4 zeigt eine abgewandelte Ausführungsform zu der Kassette gemäß Fig. 1. Auch diese in Fig. 4 dargestellte Kassette 10 dient zur Standard-Hämodialyse und zeigt weitgehend einen identischen Aufbau zu der Kassette 10 gemäß Fig. 1. Insofern erübrigt sich eine detaillierte Beschreibung der bereits beschriebenen Bestandteile der Kassette 10. Allerdings ist anstelle des Handgriffs 70 in der Ausführungsform gemäß Fig. 1 ein Dialysator 72 seitlich in die Kassette 10 integriert, wobei hier die Überleitungen 18 und 22 zum Dialysator unmittelbar in den Dialysator einmünden. Am Dialysator, der konventionell aufgebaut sein kann, sind die Dialysatanschlüsse mit 74 und 76 bezeichnet.

In Fig. 2 ist eine Kassette 10 gezeigt, die als online-Hämodiafiltrationskassette ausgeführt ist. Aufgrund der Anordnung der verschiedenen Elemente wird deutlich, daß der Kassettengrundkörper 12 ausgeht von dem Kassettengrundkörper, wie er schon anhand des Ausführungsbeispiels für die Standard-Hämodialyse in Fig. 1 beschrieben wurde. Sämtliche Elemente, die aus dieser Konfiguration bekannt sind, finden sich in der Ausführungsvariante gemäß Fig. 2 für die online-Hämodiafiltration in gleicher Weise wieder. Insofern werden sie nicht nochmals zusätzlich erläutert. Erläutert werden allerdings diejenigen Teile, die zum Betrieb als Hämodiafiltrationskassette notwendig sind. Hierzu gehört der Substituatkonnektor 80, über den Substituatflüssigkeit in die Kanäle 28 eingespeist wird. An den Kanälen sind Substituatkanalventile 82 vorgesehen, über die die Kanäle 28 an den entsprechenden Stellen verschließbar sind. Über die Kanäle wird die Substituatsflüssigkeit in zwei parallele Pumpkammern 84, die Substituatpumpkammern bilden, geleitet. Die Substituatpumpkammern 84 entsprechen im wesentlichen den Pumpkammern für das Blut 50, wie diese zuvor bereits im einzelnen beschrieben wurden. Von Kanal 28 ausgehend wird die Substituatflüssigkeit durch einen Substituattunnel 86, der auf der gegenüberliegenden Seite des Kassettengrundkörpers 12 liegt, geleitet. Der Substituattunnel ist rückseitig geeignet verschlossen, z. B. mit einer angeschweißten Folie. Die Substituatflüssigkeit 86 kann über einen Port für Predilution 88 oder über einen Port für Postdilution 90 in den das Blut führenden Kanal 28 eingeleitet werden. Die Ports sind hier wieder als Phantomventile ausgestaltet, wobei auch hier wieder auf die konstruktive Ausgestaltung entsprechend der deutschen Patentanmeldung DE 100 53 441 verwiesen wird.

Der im wesentlichen durch die Substituatpumpkammern 84 gebildete Substituatbereich wird von einem Substituatabschweißrand 92 umgeben, mit dem die Deckfolie 14 dichtend verschweißt ist, so daß dieser Substituat verarbeitende Bereich der Kassette 10 von dem blutführenden Bereich abgetrennt ist.

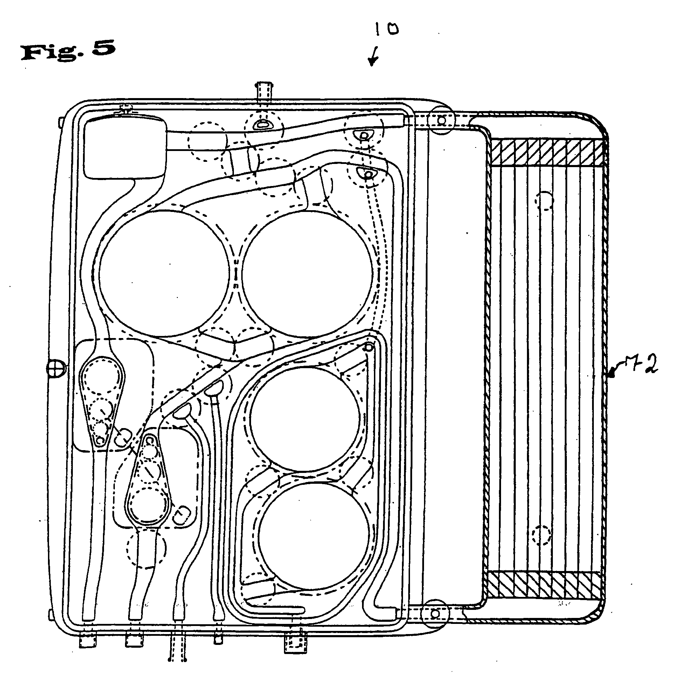

In Fig. 5 ist eine Modifikation der Ausführungsvariante gemäß Fig. 2 gezeigt. Auch hier ist wieder, ähnlich wie in der Ausführungsvariante gemäß Fig. 4 ein Dialysator 72 unmittelbar in die Kassette 10 integriert.

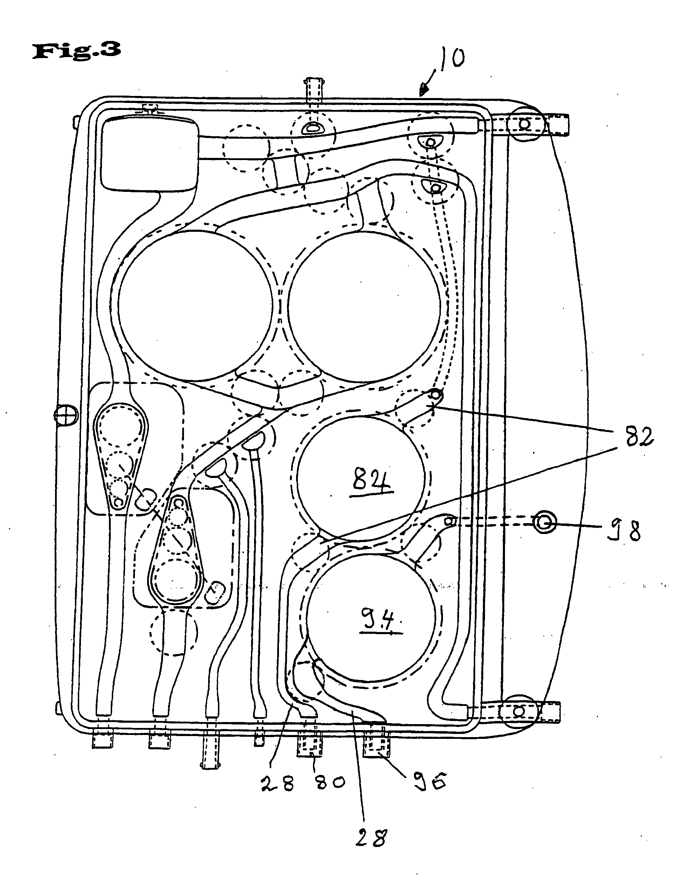

In der Fig. 3 ist als weitere integrierte Ausführungsform der Kassette eine Kassette 10 für die Akut-Behandlung dargestellt. Diese ist im Bereich des Blutbehandlungsteils identisch zu der Ausführungsvariante gemäß der Fig. 1 aufgebaut. Hinsichtlich des Substituatteils entspricht sie teilweise der Ausführungsform gemäß der Fig. 2, wobei hier nur eine Substituatpumpkammer 84 vorgesehen ist, die von der über den Substituatkonnektor 80 und den Kanal 28 zugeleiteten Substituatflüssigkeit gespeist wird. Vor und hinter der Substituatpumpkammer 84 sind hier ähnlich wie in der Ausführungsvariante gemäß Fig. 2 Substituatkanalventile 82 vorgesehen. Die weitere Pumpkammer, die in der hier vorliegenden Ausführungsvariante für die Akut-Behandlung mit 94 bezeichnet ist, ist über einen Kanal 28 mit einem Filtratausgang 96 verbunden und mündet in einen Filtratanschluß 98, der mit dem hier nicht näher dargestellten Dialysator verbunden ist.

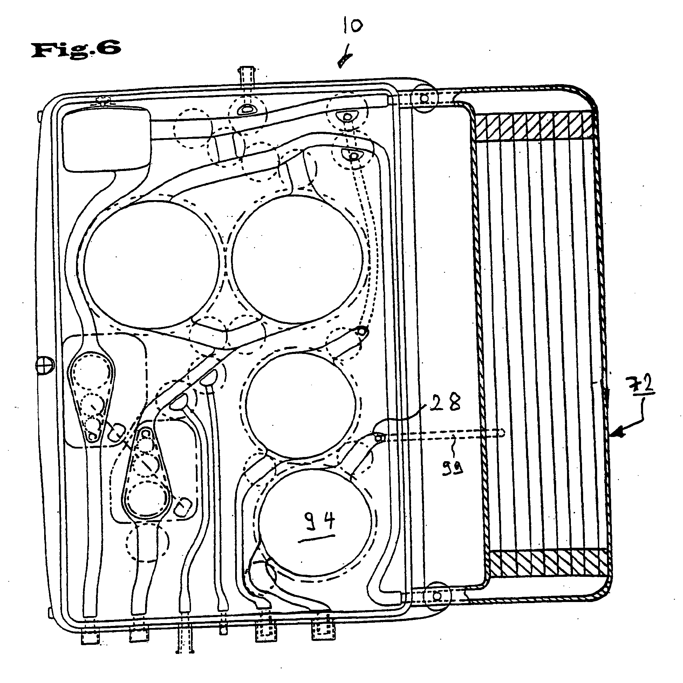

In Fig. 6 wiederum ist eine modifizierte Ausführungsvariante der Kassette 10 gemäß Fig. 3 dargestellt. Hier ist an der Stelle des Handgriffs wiederum ein Dialysator 72 integriert, wobei hier eine Verbindung 99 zwischen dem Dialysator 72 und dem das Filtrat führenden Kanal 28, der zur Filtratpumpe 94 führt, vorgesehen ist.

In Fig. 7 ist eine Ausführungsform der Flüssigkeitsbehandlungsmaschine 100 ohne eingesetzte Kassette 10 gezeigt. Diese Flüssigkeitsbehandlungsmaschine 100 ist derart aufgebaut, daß sämtliche vorgenannten Kassetten einsetzbar sind, wobei durch entsprechende Programmauswahl beispielsweise bei Einsatz der Kassette gemäß der Ausführungsvariante nach Fig. 1 ein Basis-extrakorporaler-Blutkreislauf, d. h. eine Standard-Dialyse unter Verwendung eines externen Dialysators durchgeführt wird. Bei Einsatz einer Kassette 10 gemäß dem Ausführungsbeispiel nach Fig. 2 wird beispielsweise eine online-Hämodiafiltration oder eine online-Hämofiltrationsvariante durch Einsatz der hierfür benötigten Komponenten mit ggf. automatischen Anschlüssen (nicht gezeigt) an den Fluidkreislauf des Basisgerätes verwirklicht. Auch sind hochintegrierte Varianten mit integriertem Dialysator und einer automatischen Dialysatorkonnektion möglich, wie sie seitens der Kassette in den Ausführungsvarianten gemäß der Figuren 4 und 5 gezeigt sind. Eine Akut-Dialysebehandlung ist bei Einsatz einer Kassette 10 gemäß dem Ausführungsbeispiel nach Fig. 3 möglich.

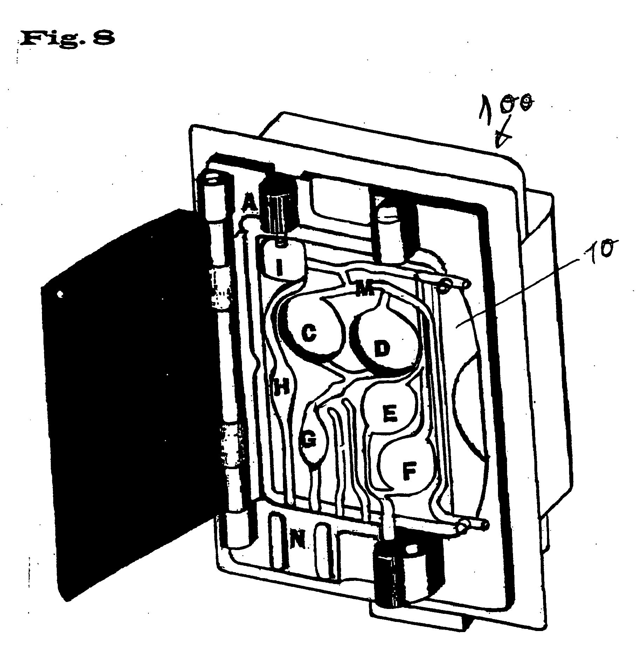

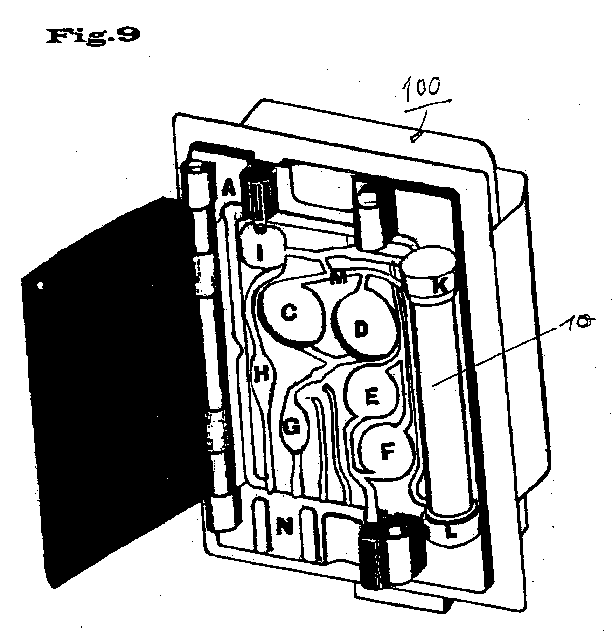

Die Flüssigkeitsbehandlungsmaschine 100 besteht im wesentlichen aus einem Rahmen 104, der die wichtigsten Komponenten umgibt bzw. enthält oder aufnimmt. Am Rahmen 104 ist einerseits eine Tür 106 angeschlagen und andererseits ist im Rahmen der Maschinenblock 108 geführt. Mittels des Rahmens 104 werden alle zwischen Tür 106 und Geräteinnerem auftretenden Kräfte aufgenommen, nämlich Türscharnier, Türverriegelung, Verpressaktorik und die Rückwand. Des weiteren enthält der Rahmen die Verriegelung der Tür 110. Zwischen der Tür 106 und dem Maschinenblock 108 wird die Kassette 10 aufgenommen, wie in den Figuren 8 und 9 gezeigt und durch Verpressung gedichtet. Im Kassettenbereich der Maschine sind Sensorikelemente enthalten, die feststellen, ob eine Kassette in der Flüssigkeitsbehandlungsmaschine richtig positioniert ist. Diese oder weitere Sensorikelemente können so ausgelegt sein, daß sie zur Erkennung des Kassettentyps geeignet sind (z. B. mit Hilfe eines Bar-Codes auf der Kassette).

Im Maschinenblock 108 sind die wesentlichen Elemente zur Ansteuerung und Kontrolle des extrakorporalen Blutkreislaufs, wie Pumpen, Ventile, und die Sensorik etc. enthalten. Dieser Maschinenblock 108 stellt die wichtigste Schnittstelle zur Kassette 10 her. Die Kassettenoberfläche koppelt hier an das Gerät an und hierdurch erfolgt die Dichtung der Kassette 10 und damit die Festlegung der Flußwege. Der Maschinenblock ist im Rahmen beweglich geführt und fixiert die Kassette 10, wie bereits zuvor beschrieben, bis die Tür geschlossen wird.

In der Flüssigkeitsbehandlungsmaschine sind Hydraulikkolbenpumpen enthalten, die in den Figuren 7, 8 und 9 hier nicht im einzelnen dargestellt sind. Es handelt sich hier einerseits um Blutpumpen oder optionale Substituatzugabepumpen bzw. Ultrafiltratpumpen. Diese sind hydraulisch mit den Pumpkammern C, D, d. h. den Blutpumpkammern oder E, F, d. h. wahlweise den optionalen Filtratpumpkammern und/oder den optionalen Substituatpumpkammern verbunden. Weiterhin sind in der Flüssigkeitsbehandlungsmaschine 100 hier nicht näher dargestellte Kompressoren zur Erzeugung des benötigten Pneumatikdrucks (Überdruck bzw. Vakuum) enthalten. Die Flüssigkeitsbehandlungsmaschine 100 weist weiterhin in nicht näher dargestellter Art und Weise einen Pneumatik-Pufferbehälter zum Ausgleich von Druckschwankungen, eine Hauptelektronik-Box, eine Heparinspritzenpumpe und ein Blutdruck-Monitor-Modul auf.

Hervorzuheben ist eine hier ebenfalls nicht näher dargestellte Verpressaktorik an der Rückwand des Rahmens 104. Hier ist ein aufpumpbares Luftkissen integriert, das den gesamten Maschinenblock 108, der ja beweglich im Rahmen 104 gelagert ist, gegen die geschlossene Tür 106 bewegen und verpressen kann.

Weiterhin ist anstelle von einzelnen luftführenden Schläuchen eine Luftverteilerplatte am Maschinenblock 108 vorgesehen, die Hauptanschlüsse zur Pneumatik enthält und über dort integrierte Kanäle ohne wesentliche Verschlauchung Druckluft und Vakuum zu den Ventilen und Aktoren führt, wobei diese gleichzeitig den Maschinenblock zum Inneren der Flüssigkeitsbehandlungsmaschine 100 abschließt.

Es können optionale Module in der Flüssigkeitsbehandlungsmaschine 100 zur Durchführung der online-Hämodiafiltration vorgesehen sein. So kann hier ein online-Zugabeport zur automatischen Ankopplung einer Kassette 10 an den Dialysatkreislauf oder ein online-Spülport zur Rückführung von Spüllösung enthalten sein.

Zum Einlegen der Kassette 10 muß die Tür 106 geöffnet werden. Die Kassette 10 wird eingelegt und nach Positionierung der Zentriergabel mittels eines Schnapphakens an der Kassette auf der Oberfläche des Maschinenblocks fixiert.

Die der Kassette zugewandte Seite des Maschinenblocks ist mit einer hier nicht näher dargestellten weichen Elastomer-Matte belegt, die nach erfolgter Verpressung die Kassette 10 abdichtet. Eine nähere Beschreibung dieser Elastomer-Matte ist bereits in der deutschen Patentanmeldung 101 57 924 derselben Anmelderin erfolgt, auf die hier vollinhaltlich Bezug genommen wird.

Nach Schließen und Verriegeln der Tür erfolgt die Verpressung durch Aufblasen des vorgenannten Luftkissens. Beim Öffnen und Entnehmen der Kassette wird vor Öffnen der Tür die Verpressung durch Ablassen der Luft im Luftkissen wieder aufgehoben.

Um eine ausreichende Verpressung zu erzielen und ein Verkippen des Maschinenblocks durch ungleichmäßige Krafteinleitung zu vermeiden, hat das Luftkissen in etwa die Größe des Maschinenblocks bzw. der Kassette 10.

Da nun aber zwischen Luftkissen und Maschinenblock weitere Komponenten, beispielsweise Ansteuerventile bzw. die Luftverteilerplatte mit den Ansteuerventilen, liegen, erfolgt die Kraftübertragung mittels Abstandsbolzen.

Der Kraftschluß zwischen Tür 106, Rahmen 104 und Rückwand erfolgt durch das Türscharnier, die Verriegelung 110 und hier nicht näher dargestellte Verbindungsbolzen zwischen Rahmen und Rückwand.

Wie bereits erwähnt muß zum einwandfreien Betrieb eine ständige Verpressung der Kassette 10 erfolgen. Hierzu ist es notwendig, daß die Tür während der Behandlung verriegelt ist. Diese Verriegelung erfolgt über zwei Verriegelungsbolzen (hier nicht näher dargestellt) am oberen rechten und unteren rechten Türbereich, wobei diese bei Betätigung, die automatisch erfolgt, in zwei entsprechende Bohrungen innerhalb der Tür 106 einfahren. Das Ein- und Ausfahren erfolgt pneumatisch. Durch die bei in der Tür eingefahrenen Bolzen und die durch die Druckbelastung der Tür auftretenden Querkräfte ist eine fehlerhafte Öffnung der Tür bei Ausfall der Pneumatik ausgeschlossen. Zur Kontrolle, ob die Verriegelung erfolgt ist, können Hall-Abstandsensoren integriert sein, die die Bewegung der Bolzen detektieren. Zusätzlich kann dieses Signal noch mit einer Information über die Türlage verknüpft sein, die durch einen gesonderten Sensor aufnehmbar ist. Zusätzlich kann der hier nicht näher dargestellte Verriegelungsbolzen noch eine Rastung aufweisen. Diese Verrastung besteht aus einer federbelasteten Rastkugel auf der Türseite, die in eine entsprechende Wölbung des Verriegelungsbolzens einrastet und die Tür in der entsprechenden Position halten kann. Zum vereinfachten Einrasten ist eine Einführschräge vorgesehen. Zum Öffnen der Tür aus der Rastposition heraus wird mittels einer Mechanik die hier vorhandene Rastkugel zurückgezogen.

Seitens der Flüssigkeitsbehandlungsmaschine 100 besteht der Blutkreislauf im wesentlichen aus mindestens einer hydraulisch angesteuerten Membranpumpe mit zwei unabhängigen Pumpkammern C und D, die als hochgenaue Flußpumpe oder als volumetrische Dosiereinheit eingesetzt werden können, einer Reihe von Ventilen M, O und Klemmen N zur Flußwegesteuerung, einer zur Überwachung und Steuerung notwendigen, hochintegrierten Sensorik G, H, einer aktiven Luftentfernung, d. h. einer Luftabscheidekammer I mit angeschlossener Kassette-Entlüftung A, aus dem Blutkreislauf (luftfreier Kreislauf) und einer Tür 106 zur Fixierung der Kassette.

Die Flüssigkeitsbehandlungsmaschine 100 umfaßt jeweils ein Pneumatiksystem für den Überdruck und ein Pneumatiksystem für den Unterdruck. Der Unterdruck dient beispielsweise dazu, zwischen der Folie 14 der Kassette 10 und der Geräteseite einen Unterdruck anzulegen, um bei plastischer Verformung der Folie eine Kanalverengung zu verhindern, um an Zugabestellen die Folie abzuheben und damit den Zugang freihalten zu können, um eine Luftcompliance in den Pumpeinrichtungen zu vermeiden und um an speziellen Sensorpositionen eine luftfreie Ankopplung zwischen Sensor und Folie gewährleisten zu können. Die Luftabsaugung bedingt Öffnungen in der Geräteseite und eine daran angeschlossene Absaugeinheit, d. h. eine Vakuumpumpe, wobei die Vakuumverteilung möglichst gleichmäßig und sicher über die gesamte Fläche gewährleistet sein soll. Im Ruhezustand sollen die Öffnungen zumindest weitestgehend verschlossen sein, um hier eine gute Reinigung zu ermöglichen. Im Betrieb jedoch soll eine störungsfreie Luftabsaugung ermöglicht sein. Gelöst wird diese Problematik durch die bereits zuvor erwähnte Elastomer-Matte, die in der deutschen Patentanmeldung 101 57 924 beschrieben wurde.

Bei der Kassette 10 sind bis auf den Randbereich und einige Sicherheitsabschweißungen keinerlei Kanaldichtungen enthalten. Daher muß die Abdichtung aller Flußwege und Kanäle durch Verpressung erfolgen. Hierzu weist die Kassette an den Kanalrändern Dichtwulste 52 auf, die bereits zuvor beschrieben wurden und die bei Verpressung des Disposables zwischen Maschinenblock 108 und Tür 106 durch Eindrücken in die elastische Matte abdichtbar sind.

Die hier nicht näher dargestellte Luftverteilerplatte befindet sich auf der Rückseite des Maschinenblocks 108 und ist mit den beispielsweise zwei Membranpumpen des Pneumatiksystems verbunden, nämlich der Überdruckpumpe und der Unterdruckpumpe. Die Luftverteilerplatte ist gegenüber der Maschinenblockrückseite durch eine Dichtungsmatte abgedichtet und ermöglicht die Druckluft- und Vakuumzuleitung über integrierte Kanalstrukturen, so daß nicht jedes Ventil eine eigene Verschlauchung benötigt. Auf der Luftverteilerplatte bestehen mehrere Kreisläufe, nämlich ein Vakuumkreis, ein Druckluftkreis, der direkt mit dem Kompressor verbunden ist zur Versorgung von Komponenten, die immer Druckluft benötigen, ein Druckluftkreis zum Schutz empfindlicher Komponenten, die nur zu bestimmten Zuständen mit Druckluft beaufschlagt werden dürfen, wobei dieser durch ein Schaltventil vom Kompressor abtrennbar ist und einen Abluftkreis.

Durch Integration einer Vielzahl von Ansteuerventilen auf der Luftverteilerplatte kann auch die elektrische Versorgung über eine kleine Ansteuerplatine zusammenfaßbar sein. Da mehrere Ventile nur bei bestimmten Optionen benötigt werden, muß eine modulare Nachrüstbarkeit gewährleistet sein.

Die Sensorik und die Pumpanschlüsse werden durch Ausbrüche und Aussparungen durch die Platte hindurchgeführt.

Zur Überwachung und Steuerung des extrakorporalen Blutkreislaufs sind Sensoren notwendig, die in der vorliegenden Flüssigkeitsbehandlungsmaschine 100 in integrierten Sensormodulen zusammengefaßt sind. Jeweils zwei Module arbeiten als Paar zusammen. Dabei ist ein Modul in der Tür 106 und das Gegenstück im Maschinenblock 108 untergebracht. Überwacht werden sowohl der arterielle Zweig durch die arterielle Meßkammer G und der venöse Zweig durch die venöse Meßkammer H. Die integrierte Meßsensorik wird ausführlich in den deutschen Patentanmeldungen DE 198 37 667 A und DE 101 43 137 der gleichen Patentanmelderin beschrieben. Zusammen haben die Sensoren u. a. folgende Eigenschaften bzw. bieten die folgenden Möglichkeiten:
- Messung und Kontrolle des Blutvolumens,
- Messung des Hämatokrits,
- Messung und Kontrolle der thermischen Energiebilanz,
- Messung und Kontrolle der Körpertemperatur,
- Messung der Verhältnisse der Fistel (mit Zirkulation),
- Luftdetektion,
- Fisteldruckmessung.

Ein Multisensormodul ist üblicherweise bestückt mit einem Ultraschallsensor zur Volumenkontrolle, Messung des Hämatokrits und der Luftdetektion, einem Temperatursensor für die automatische Zugangsanalyse, Körpertemperaturkontrolle und thermische Energiebilanz, einem Drucksensor zur Drucküberwachung und einem optischen Sensor zur automatischen Erkennung von Blut.

Hinsichtlich der Ventile M und der Pumpventile O sei hinsichtlich ihres Aufbaus nochmals auf die DE 100 46 651 A1 verwiesen.

Neben den zuvor genannten Ventilen, die in Figur 7 dargestellt sind, sind zusätzlich sogenannte Phantomvenile, die in dieser Figur 7 nicht näher eingezeichnet sind vorhanden. Hinsichtlich des Aufbaus und der Funktionsweise der Phantomventile kann auf die DE 100 53 441 A1 verwiesen werden.

Mit N sind Sicherheitsklemmen bezeichnet, die zur Erzielung eines sicheren Zustandes während eines Alarms im extrakorporalen Blutkreislauf dienen, wobei sie die Patientenleitung und damit jeglichen Blutfluß vom bzw. zum Patienten unterbrechen. Zur Vermeidung unerwünschter Compliance-Effekte und da das System für eine Flußumkehr ausgelegt ist, muß diese Sicherheitsfunktion sowohl auf arterieller wie auch auf venöser Seite gewährleistet sein, so daß zwei Absperrklemmen N zum Einsatz kommen, die mechanisch gekoppelt sein können.

Die Absperrklemmen sollen möglichst nah am Patienten wirksam werden, um jeglichen Störeinfluß minimieren zu können und hohen Sicherheitsanforderungen gerecht zu werden. Daher werden Schlauchklemmen, die direkt auf die Patientenschläuche wirken, eingesetzt.

Eine mögliche Ausführungsform, wie sie hier vorgesehen ist, besteht in der Abklemmung der Schläuche gegen eine Abklemmleiste auf der Türinnenseite mittels eines wiederschließenden pneumatisch geöffneten Klemmstößels. Ein derartiges System ist passiv federschließend, nämlich drucklos und stromlos und damit auch im Fall eines Ausfalls unter Sicherheitsgesichtspunkten vorteilhaft.

In der Fig. 8 ist eine Flüssigkeitsbehandlungsmaschine 100 entsprechend der Fig. 7 mit einer eingesetzten Kassette 10 entsprechend der Fig. 2 dargestellt. In Fig. 9 dagegen ist eine Flüssigkeitsbehandlungsmaschine 100 mit einer Kassette 10 entsprechend der Ausführungsvariante gemäß Fig. 5 gezeigt, wobei hier in der Kassette der Dialysator mit der Flüssigkeitsbehandlungsmaschine 100 eine automatische Dialysatkonnektion K und L aufweist.

Die hier vorgestellte neue Vorrichtung verfolgt einen strikt modularen Ansatz unter Erzielung einer hohen Flexibilität und Einsatzmöglichkeit im Hinblick auch auf zukünftige Einsatzmöglichkeiten und Optionen. Das integrierte Blutmodul ermöglicht die Durchführung des gesamten Bereichs der Blutbehandlungsverfahren, nämlich die Standard-Hämodialyse, die online-Hämodiafiltration, die online-Hämofiltration und auch die Akut-Behandlung.

Hinsichtlich der Akut-Behandlung ist darauf zu verweisen, daß die Maschinen, die zur Akut-Behandlung, d. h. zur Akut-Dialyse bzw. Akut-Filtration dienen, einfach aufgebaut sein müssen, um entsprechend leicht transportiert und ohne eine komplexe Versorgungsstruktur (z. B. Wasseranschluß) funktionieren zu können. Bei diesem System wird daher praktisch ausnahmslos mit Beuteln mit vorgefertigten Lösungen gearbeitet. Mit der in den Figuren 3 bzw. 6 gezeigten Ausführungsformen kann dann leicht eine Akut-Hämofiltration durchgeführt werden, bei der Substituat aus einem Beutel zugeführt und Filtrat aus dem Filter in einen leeren Beutel mit den gezeigten Pumpen entfernt wird. Bis auf den Anschluß der Beutel ist in diesem Fall keine weitere Maßnahme notwendig. Es wäre natürlich dennoch möglich, mit entsprechendem Aufwand zusätzlich eine Dialyse zu ermöglichen. Desweiteren könnte alternativ die Substituatpumpe als Dialysatzuführpumpe Verwendung finden, wenn die Anschlüsse innerhalb der Kassette entsprechend verändert würden. Dann könnte in Beuteln abgefüllte Dialysierflüssigkeit über die Membranpumpe bilanziert dem Filter zugeführt werden, während über die Filtratpumpe gesteuert Flüssigkeit abgeführt wird. Auch bei einer solchen Maschine wären keine weiteren Komponenten zur Flüssigkeitssteuerung notwendig.

Jede dieser Behandlungsarten kann sowohl im Doppelnadel- sowie im Einzelnadelmodus erfolgen. Hinsichtlich der Beschreibung des Doppelnadel- bzw. Einzelnadelmodus sei hier auf das deutsche Patent DE 100 42 324 C1 verwiesen.

In der hier dargestellten Erfindung ist eine Reihe von Neuentwicklungen umfasst, wie die neuartige Gestaltung der Pumpkammer sowie der dazugehörigen maschinenseitigen Pumpkalotte als auch die Luftabscheidekammer. Für diese Bestandteile bzw. Kassetten bzw. Maschinen zur Blutbehandlung wird im Rahmen der Erfindung isoliert, das heißt ohne Zusammenwirkung mit anderen Elementen der Kassetten oder Maschinen Schutz beansprucht.

## Patentansprüche

1. Vorrichtung zur Behandlung einer medizinischen Flüssigkeit mit einer Flüssigkeitsbehandlungsmaschine (100) und einer in diese einsetzbaren Kassette (10) im Wesentlichen bestehend aus einem starren Kassettengrundkörper (12) mit eingelassenen Kammern und Kanälen und einer diese abdeckenden Folie (14),
**dadurch gekennzeichnet**,
dass wenigstens eine der Kammern der Kassette (10) eine Pumpkammer (50) zum Pumpen von Blut ist und dass die mindestens eine Pumpkammer (50) im wesentlichen die Form eines Kugelabschnittes hat und dass um die Kammer herum am Rand ein hochstehender Wulst (52) ausgeformt und der umlaufende Rand des Kugelabschnittes etwas tiefer gesetzt ist, so dass sich in der Ausdrückphase zwischen dem oberen Rand des Kassettengrundkörpers (12) und der Deckfolie ein Spülkanal (54) ausbildet.

2. Vorrichtung nach Anspruch 1, **dadurch gekennzeichnet, dass** die mindestens eine Pumpkammer (50) über im wesentlichen tangentiale Zu- und Abgänge verfügt.

3. Vorrichtung nach Anspruch 1 oder 2, **dadurch gekennzeichnet, dass** die Pumpkammern (50) mittels maschinenseitig vorgesehener Membranpumpen aktivierbar sind.

4. Vorrichtung nach Anspruch 3, **dadurch gekennzeichnet, dass** die Membranpumpen eine Pumpkalotte aufweisen.

5. Vorrichtung nach Anspruch 4, **dadurch gekennzeichnet, dass** die maschinenseitige Pumpkalotte einen kleineren Radius verglichen zum Radius der kassettenseitigen Pumpkammer (50) aufweist, so dass der Spülkanal (54) der Breite des Radiusunterschieds zwischen der Pumpkalotte und der Pumpkammer entspricht.

6. Vorrichtung nach Anspruch 5, **dadurch gekennzeichnet, dass** der Spülkanal (54) ein Ringraum in der Ausdrück-Extremstellung ist.

7. Vorrichtung nach einem der vorhergehenden Ansprüche, **dadurch gekennzeichnet, dass** zwei Pumpkammern (50) zum Pumpen des Blutes dienen bzw. vorgesehen sind.

8. Vorrichtung nach einem der vorhergehenden Ansprüche, **dadurch gekennzeichnet, dass** in der Flüssigkeitsbehandlungsmaschine (100) Aktoren und Sensoren zum Betrieb der Vorrichtung mit der eingesetzten Kassette (10) derart angeordnet sind, dass Kassetten (10) in verschiedenen Integrationsformen einsetzbar sind.

9. Kassette (10) zum Einsatz in einer Vorrichtung nach einem der Ansprüche 1 bis 8, **dadurch gekennzeichnet, dass** die Kassette (10) im wesentlichen aus einem starren Kassettengrundkörper (12) mit eingelassenen Kammern und Kanälen und einer diese abdeckenden Folie (14) besteht und dass wenigstens eine der Kammern der Kassette eine Pumpkammer (50) zum Pumpen von Blut ist und dass die mindestens eine Pumpkammer (50) im wesentlichen die Form eines Kugelabschnittes hat und dass um die Kammer herum am Rand ein hochstehender Wulst (52) ausgeformt und der umlaufende Rand des Kugelabschnittes etwas tiefer gesetzt ist, so dass sich in der Ausdrückphase zwischen dem oberen Rand des Kassettengrundkörpers (12) und der Deckfolie (14) ein Spülkanal (54) ausbildet.

10. Kassette (10) nach Anspruch 9, **dadurch gekennzeichnet, dass** sie mindestens eine, vorzugsweise zwei Pumpkammern (50), mindestens eine Meßkammer, eine Entlüftungskammer, integrierte Kanäle und mehrere Ports aufweist.

11. Kassette (10) nach einem der Ansprüche 9 oder 10, **dadurch gekennzeichnet, dass** die mindestens eine Meßkammer die Form einer Diffusordüse aufweist.

12. Kassette (10) nach einem der Ansprüche 9 bis 11, **dadurch gekennzeichnet, dass** alle Kanäle und Kammern jeweils seitlich einen solchen Randwulst (52) aufweisen, der der Deckfolie (14) zugewandt ist.

13. Kassette (10) nach Anspruch 12, **dadurch gekennzeichnet, dass** auf der Kanalrückseite zusätzlich kanalbegleitende Stege ausgebildet sind, über die eine linienhafte Anpresskraftverteilung erreichbar ist.

14. Kassette (10) nach einem der Ansprüche 9 bis 13, **dadurch gekennzeichnet, dass** der Spülkanal (54) ein Ringraum in der Ausdrück-Extremstellung ist.

15. Kassette (10) nach einem der Ansprüche 9 bis 14, **dadurch gekennzeichnet, dass** zwei Pumpkammern (50) zum Pumpen des Blutes dienen bzw. vorgesehen sind.

16. Kassettenset bestehend aus Kassetten (10) nach mindestens einem der Ansprüche 9 bis 15, **dadurch gekennzeichnet, dass** das Set Kassetten (10) für die Anwendungszwecke Standard-Hämodialyse, online-Hämodiafiltration oder online-Hämofiltration, und Akutbehandlung umfasst.

## Claims

1. An apparatus for the treatment of a medical liquid comprising a liquid treatment machine (100) and a cassette (10) insertable into it substantially consisting of a rigid base body (12) of a cassette with fitted chambers and passages and a foil (14) covering them,
**characterized in that**
at least one of the chambers of the cassette (10) is a pump chamber (50) for pumping blood and **in that** the at least one pump chamber (50) substantially has the shape of a spherical section and **in that** a standing bead (52) is formed at the edge around the chamber and the circumferential edge of the spherical section is set slightly lower such that a flushing passage (54) is formed between the upper edge of the base body (12) of the cassette and the cover foil in the pressing-out phase.

2. An apparatus in accordance with claim 1, wherein the at least one pump chamber (50) has substantially tangential inlets and outlets.

3. An apparatus in accordance with either of claims 1 or 2, wherein the pump chambers (50) can be activated by membrane pumps provided at the machine side.

4. An apparatus in accordance with claim 3, wherein the membrane pumps have a spherical pump surface.

5. An apparatus in accordance with claim 4, wherein the spherical surface of the pump at the machine side has a smaller radius in comparison with the radius of the pump chamber (50) at the cassette side such that the flushing passage (54) corresponds to the width of the radius difference between the spherical surface of the pump and the pump chamber.

6. An apparatus in accordance with claim 5, wherein the flushing passage (54) is an annulus in the extreme press-out position.

7. An apparatus in accordance with any one of the preceding claims, wherein two pump chambers (50) serve to pump blood or are provided to pump blood.

8. An apparatus in accordance with any one of the preceding claims, wherein actuators and sensors are arranged in the liquid treatment machine (100) for the operation of the apparatus with the inserted cassette (10) such that cassettes (10) can be inserted in different integration shapes.

9. A cassette (10) for use in an apparatus in accordance with any one of claims 1 to 8, wherein the cassette (10) substantially consists of a rigid base body (12) of a cassette with fitted chambers and passages and a foil (14) covering them and wherein at least one of the chambers of the cassette is a pump chamber (50) for pumping blood and wherein the at least one pump chamber (50) substantially has the shape of a spherical section and wherein a standing bead (52) is formed at the edge around the chamber and the circumferential edge of the spherical section is set slightly lower such that a flushing passage (54) is formed between the upper edge of the base body (12) of the cassette and the cover foil (14) in the pressing-out phase.

10. A cassette (10) in accordance with claim 9, wherein it has at least one, preferably two, pump chambers (50), at least one measuring chamber, a venting chamber, integrated passages and a plurality of ports.

11. A cassette (10) in accordance with either of claims 9 or 10, wherein the at least one measuring chamber has the shape of a diffuser nozzle.

12. A cassette (10) in accordance with any one of claims 9 to 11, wherein all passages and chambers each have such an edge bead (52) at the side which faces the cover foil (14).

13. A cassette (10) in accordance with claim 12, wherein webs accompanying the passage are formed additionally at the rear side of the passage and a linear pressing force distribution can be achieved via them.

14. A cassette (10) in accordance with any one of the claims 9 to 13, wherein the flushing passage (54) is an annulus in the extreme press-out position.

15. A cassette (10) in accordance with any one of the claims 9 to 14, wherein two pump chambers (50) serve to pump blood or are provided to pump blood.

16. A cassette set consisting of cassettes (10) in accordance with at least one of claims 9 to 15, wherein the set comprises cassettes (10) for the application purposes standard hemodialysis, online hemodiafiltration or online hemofiltration and acute treatment.

## Revendications

1. Dispositif de traitement pour traiter un fluide médical comprenant une machine de traitement de fluide (100) et une cassette (10) insérable dans celle-ci qui est composée essentiellement d'un corps de base de cassette (12) rigide avec des chambres et des canaux encastrés et un film (14) les recouvrant,
**caractérisé en ce que**
au moins une des chambres de la cassette (10) est une chambre de pompage (50) destinée à pomper du sang et **en ce que** l'au moins une chambre de pompage (50) présente essentiellement la forme d'un segment sphérique et **en ce qu'**un renflement (52) saillant vers le haut est formé au bord autour de la chambre et **en ce que** le bord périphérique du segment sphérique est placé légèrement plus bas, de sorte que dans la phase d'éjection un canal de rinçage (54) se forme entre le bord supérieur du corps de base de cassette (12) et le film de recouvrement.

2. Dispositif selon la revendication 1, **caractérisé en ce que** l'au moins une chambre de pompage (50) dispose d'entrées et de sorties essentiellement tangentielles.

3. Dispositif selon la revendication 1 ou 2, **caractérisé en ce que** les chambres de pompage (50) peuvent être activées au moyen de pompes à diaphragme prévues côté machine.

4. Dispositif selon la revendication 3, **caractérisé en ce que** les pompes à diaphragme comportent une calotte de pompe.

5. Dispositif selon la revendication 4, **caractérisé en ce que** la calotte de pompe côté machine présente un rayon plus petit comparé au rayon de la chambre de pompage (50) côté cassette, de sorte que le canal de rinçage (54) correspond à la largeur de la différence de rayon entre la calotte de pompe et la chambre de pompe.

6. Dispositif selon la revendication 5, **caractérisé en ce que** le canal de rinçage (54) est un espace annulaire dans la position extrême d'éjection.

7. Dispositif selon l'une des revendications précédentes, **caractérisé en ce que** deux chambres de pompage (50) servent à ou sont destinées à pomper le sang.

8. Dispositif selon l'une des revendications précédentes, **caractérisé en ce que** dans la machine de traitement de fluide (100) des actionneurs et des capteurs pour le fonctionnement du dispositif avec la cassette (10) insérée sont disposés de telle manière que des cassettes (10) de différentes formes d'intégration sont utilisables.

9. Cassette (10) destinée à être utilisée dans un dispositif selon l'une des revendications 1 à 8, **caractérisée en ce que** la cassette (10) est composée essentiellement d'un corps de base de cassette (12) rigide avec des chambres et des canaux encastrés et d'un film (14) les recouvrant et **en ce qu'**au moins l'une des chambres de la cassette est une chambre de pompage (50) destinée à pomper du sang et **en ce que** l'au moins une chambre de pompage (50) présente essentiellement la forme d'un segment sphérique et **en ce qu'**un renflement (52) saillant vers le haut est formé au bord autour de la chambre et le bord périphérique du segment sphérique est placé légèrement plus bas de sorte qu'un canal de rinçage (54) se forme dans la phase d'éjection entre le bord supérieur du corps de base de cassette (12) et le film de recouvrement (14).

10. Cassette (10) selon la revendication 9, **caractérisée en ce qu'**elle comporte au moins une, de préférence deux chambres de pompage (50), au moins une chambre de mesure, une chambre de purge de l'air, des canaux intégrés et plusieurs orifices.

11. Cassette (10) selon l'une des revendications 9 ou 10, **caractérisée en ce que** l'au moins une chambre de mesure présente la forme d'une buse de diffuseur.

12. Cassette (10) selon l'une des revendications 9 à 11, **caractérisée en ce que** tous les canaux et toutes les chambres comportent sur le côté respectivement un renflement de bord (52) de ce type qui est orienté vers le film de recouvrement (14).

13. Cassette (10) selon la revendication 12, **caractérisée en ce que** sur la face arrière du canal, des nervures longeant le canal sont formées en plus, par le biais desquelles une distribution de la force de pression peut être obtenue.

14. Cassette (10) selon l'une des revendications 9 à 13, **caractérisée en ce que** le canal de rinçage (54) est un espace annulaire dans la position extrême d'éjection.

15. Cassette (10) selon l'une des revendications 9 à 14, **caractérisée en ce que** deux chambres de pompage (50) servent à ou sont destinées à pomper le sang. '

16. Ensemble cassette composé d'une cassette (10) selon au moins l'une des revendications 9 à 15, **caractérisé en ce que** l'ensemble comprend des cassettes (10) destinés aux applications d'hémodialyse standard, d'hémodiafiltration en ligne ou d'hémofiltration en ligne, et de traitement aigu.
